**Europäisches Patentamt**

**European Patent Office**

**Office européen des brevets**

(11) Publication number : **0 437 524 B1**

# EUROPEAN PATENT SPECIFICATION

(45) Date of publication of patent specification :
**15.04.92 Bulletin 92/16**

(51) Int. Cl.[5] : **C11C 3/08,** A61K 31/23

(21) Application number : **89911785.7**

(22) Date of filing : **10.10.89**

(86) International application number :
**PCT/DK89/00237**

(87) International publication number :
**WO 90/04011 19.04.90 Gazette 90/09**

(54) **TRIGLYCERIDE AND NUTRITIONAL COMPOSITION COMPRISING SUCH TRIGLYCERIDES.**

(30) Priority : **10.10.88 DK 5652/88**

(43) Date of publication of application :
**24.07.91 Bulletin 91/30**

(45) Publication of the grant of the patent :
**15.04.92 Bulletin 92/16**

(84) Designated Contracting States :
**AT BE CH DE FR GB IT LI LU NL SE**

(56) References cited :
**WO-A-88/09325**
**US-A- 4 607 052**
**US-A- 4 701 468**
**US-A- 4 701 469**

(73) Proprietor : **NOVO NORDISK A/S**
**Novo Allé**
**DK-2880 Bagsvaerd (DK)**

(72) Inventor : **HANSEN, Tomas, Tage**
**Tonedraget 5**
**DK-3450 Alleroed (DK)**
Inventor : **GODTFREDSEN, Sven, Erik**
**Smedegade 15B**
**DK-3500 Vaerloese (DK)**

(74) Representative : **Bach, Niels et al**
**c/o Novo Nordisk A/S Patent Dept. Novo Allé**
**DK-2880 Bagsvaerd (DK)**

**Description**

The invention relates to triglycerides and a nutritional composition comprising such triglycerides.

Arachidonic acid is an extremely important fatty acid which is involved in vital physiological processes. In man arachidonic acid is generated from the essential fatty acid linoleic acid which, by the action of a desaturase enzyme, is converted into γ-linolenic acid and subsequently into dihomo-γ-linolenic acid and finally into arachidonic acid. The importance of arachidonic acid is due to its role as a precursor of the prostaglandins which are involved in key events in the body where they regulate e.g. the tonus of blood vessels (and thus blood pressure) or the tonus of the smooth muscles in the bronchial walls and thus the respiratory distress in conditions as e.g. asthma.

It is well known that the biosynthesis of arachidonic acid described above is less efficient in elderly people which produce small amounts only of the desaturase enzyme mentioned and also that many foods contain insufficient amounts of the arachidonic acid precursors. As a consequence, people of all ages may suffer from a shortage of arachidonic acid due to improper nutrition. Finally, in the diseased state there is a high demand of arachidonic acid due to the involvement of the prostaglandins in central defense and repair mechanisms of the body. Therefore, diseased people often suffer from a shortage of arachidonic acid which impart recovery and survival.

As a consequence of the above mentioned and other roles of arachidonic acid as well there is a need for highly efficient methods for supplying arachidonic acid in enteral as well as in parenteral nutrition. Since fatty acids are best incorporated into parenteral and enteral nutritional products in the form of triglycerides there is, accordingly, a need for triglycerides which contain arachidonic acid in a fashion which ensures that the compound is optimally available for the body.

However, it is a well known fact, that triglycerides containing long chain fatty acids only (e.g. arachidonic acid) are absorbed more slowly than the corresponding triglycerides containing medium chain length acids only ($C_8$-$C_{10}$) (Metabolism, Vol. 38, p. 507-513, 1989). Furthermore it is known that the human lipases, especially human pancreatic lipase, have a low activity towards polyunsaturated acids (e.g. arachidonic acid) (Lipids, Vol. 22, 711-714, 1987) and e.g. towards ω-6 fatty acids. This is the case also for the lipases as e.g. the lipoprotein lipase involved in cleavage of triglycerides applied as emulsions in parenteral nutrition. Hitherto the only useful sources of arachidonic acid for enteral nutrition of the general public and e.g. diseased patients or for use in parenteral nutrition of e.g. diseased patients have been mixtures of various arachidonic acid containing natural fats and oils. Since these raw materials contain mainly long chain fatty acids they are relatively poor as energy substrates and they provide the essential fatty acids as e.g. arachidonic acid in a poor bioavailable state when applied in enteral as well as in parenteral nutrition.

A very important clinical aspect in regard to the bioavailability is the slower metabolism in comparison to emulsions containing MCT, which are known to cause metabolic acidosis, when infused intravenously.

Thus, the purpose of the present invention is the provision of triglycerides which are devoid of the problems mentioned above regarding arachidonic acid containing triglycerides and which can thus be formulated as an enterally administrable composition which exhibits an improved bioavailability in regard to arachidonic acid, or which can be formulated as a parenterally administrable composition, which exhibits an improved bioavailability in regard to arachidonic acid besides being an efficient energy source.

The triglycerides according to the invention are 2-arachidoyl-1,3-di(octanoyl/decanoyl) glycerol. It is to be understood that this term encompasses both the pure 1,3-dioctanoyl triglyceride, the pure 1,3-didecanoyl triglyceride, and furthermore the 1-octanoyl-3-decanoyl triglyceride and the 1-decanoyl-3-octanoyl triglyceride, the 2-position of course in all cases being occupied by arachidonic acid. Surprisingly it has been found that these triglycerides give rise to an improved bioavailability of arachidonic acid in enteral as well as in parenteral nutritional products as compared to conventional sources of arachidonic acid used in nutritional products.

Also, it has been found that the enterally administrable composition according to the invention can be better tolerated and digested and exhibits a better smell and a more optimal nutrition than the previoulsy known sources of arachidonic acid. Thus, the enterally administrable composition according to the invention exhibits a vastly reduced tendency to diarrhoeas. Moreover, the triglycerides of the invention turn out, surprisingly, to be very efficient sources of energy in enteral as well as in parenteral nutrition - the arachidonic acid moiety of the triglycerides being in an optimal position in the molecule in respect to their cleavage by lipoprotein lipase. Also, the triglycerides of the invention appear to allow preservation of the arachidonic acid in a hydrophobic form which can pass the intestinal mucosal layer and reach the enteroside in an optimal form for further esterification, lipoprotein transport and clearance.

Surprisingly, the triglycerides of the invention appear to exhibit physical properties which allow facile formulation of the compounds in liquid products as well as in powdered products exhibiting excellent wettability properties. In the liquid form the products of the invention possess excellent stabilities making sterilization of e.g. parenteral products containing the triglycerides of the invention reliable, easy and safe.

The triglycerides of the invention allow formulation of enteral feeding products which give rise to arachidonic acid level close to the optimally required levels making unnecessary use of large amounts of natural fat and oil products containing arachidonic acid which are sensitive to oxidation and polymerisation processes. Detrimental processes of polyunsaturated fats and oils leading to negative nutritional forms are thus commonly associated with gastric and intestinal problems due to oxidation and polymerisation products of the polyunsaturated fatty acid. Such oxidized forms of arachidonic acid can interfere with nitrogen uptake by interacting with sensitive amino acids in nutritional formula. These highly undesired effects are diminished or even avoided by applying triglycerides according to the invention.

For use in parenteral feeding the triglycerides according to the invention can be incorporated into lipid emulsions where the liquid phase amounts up to 30% of the emulsion and they can be processed using the usual techniques to provide chylomicronlike particles.

The triglycerides of the invention are advantageously applied in such emulsions due to their fast conversion by lipoprotein lipase and endothelial lipase and the consequential avoidance of the discomfort and side effects of lipolipaedemia. Arachidonic acid applied in triglycerides of the invention are thus cleared quickly and efficiently thereby providing the essential fatty acid concomitantly with short chain acids useful as energy substrates.

The use of triglycerides according to the invention in parenteral nutritional products is further particularly advantageous since the relatively high polarity of the triglycerides favour the stability of their emulsions which are subjected to severe heat treatments during their manufacturing. Use of such emulsion is particularly advantageous for nutrition of severely ill patients e.g. post-operatively in which the desaturation and elongation system is insufficient to meet the needs.

A further advantage of the triglycerides of the invention is related to their solubility in other oils as e.g. vegetable oils. Such solutions can be used as such as a nutritional support. The triglycerides of the invention may also be formulated into creams and related products which allow the use of the compounds for topical treatment of e.g. skin diseases associated with essential fatty acid deficiencies.

This invention is a selection invention in the sense that a general chemical formulation comprising the triglycerides according to the invention together with a very large number of other triglycerides belong to the prior art, vide FR 2515174, whereas the triglycerides according to the invention do not belong to the prior art. The triglycerides according to the invention are described and synthetized for the first time by the inventors, and also, the superior effect of the triglycerides according to the invention is demonstrated for the first time by the inventors.

Also, a general chemical formula comprising the triglycerides according to the invention appears from US 4,607,052, 4,701,468, and 4,701,469. Furthermore, it is indicated in these US patents that these triglycerides in general are useful nutrients, which can be administered in an oral, enteral and parenteral way.

A preferred embodiment of the triglycerides according to the invention is characterized by the fact that they have a purity of at least 10%, preferably at least 30%, more preferably at least 50%, even more preferably at least 75%, and most preferably at least 90%. The higher the purity of the triglycerides, the more efficient the absorption of the triglycerides.

Also, the invention comprises a nutritional composition, which comprises the triglyceride according to the invention. This composition can be either the triglycerides according to the invention without the other constituents which are necessary for a full nutritional composition, i.e. mainly vitamins, proteins and carbohydrates, or the triglycerides according to the invention together with these other constituents.

A preferred embodiment of the nutritional composition is parenterally administrable. This composition is an emulsion.

A preferred embodiment of the nutrional composition is enterally administrable. This composition is either an emulsion or an oil.

A preferred embodiment of the nutritional composition according to the invention is intended as an animal fodder. Certain carnivores do not have the ability to desaturate linoleic acid and are thus dependent on a direct supply of arachidonic acid rather than of arachidonic acid precursors. This applies e.g. to felines as cats, lions and other animals. Usually these animals obtain their arachidonic acid by eating meat but in today's society where meat substitutes find increasing use for feeding of animals there is a need for materials which can be incorporated into animal food and ensure an optimal nutrition of the animals in regard to arachidonic acid. The products of the invention are well suited for this purpose.

A preferred embodiment of the enteral composition according to the invention is in fluid form. The enteral composition can be an emulsion or a pure oil.

A preferred embodiment of the enteral composition according to the invention is in powder form, whereby the triglycerides are encapsulated or microencapsulated. One of the manners, in which the droplets of triglyceride can be encapsulated, is described in Danochemo Technical Information on microencapsulated Product, Malmparken 5, 2750 Ballerup, Denmark.

**Claims**

1. The triglycerides 2-arachidoyl-1,3-di(octanoyl/-decanoyl) glycerol.

2. Triglycerides according to Claim 1, which have a purity of at least 10%, preferably at least 30%, more preferably at least 50%, even more preferably at least 75%, and most preferably at least 90%.

3. Nutritional composition, which comprises the triglycerides according to Claim 1 or 2.

4. Nutritional composition according to Claim 3, which is parenterally administrable.

5. Nutritional composition according to Claim 3, which is enterally administrable.

6. Nutritional composition according to Claim 5, intended as animal fodder.

7. Nutritional composition according to Claim 5, which is in fluid form.

8. Nutritional composition according to Claim 5, which is in powder form, whereby the triglycerides are encapsulated or microencapsulated.

**Patentansprüche**

1. Die Triglyceride 2-Arachidoyl-1,3-di(octanoyl/decanoyl)glycerin.

2. Triglyceride nach Anspruch 1, dadurch gekennzeichnet, daß sie eine Reinheit von wenigstens 10 %, vorzugsweise wenigstens 30 %, bevorzugter wenigstens 50 %, noch bevorzugter wenigstens 75 % und am bevorzugtesten wenigstens 90 % besitzen.

3. Nahrungszusammensetzung, dadurch gekennzeichnet, daß sie die Triglyceride nach Anspruch 1 oder 2 umfaßt.

4. Nahrungszusammensetzung nach Anspruch 3, dadurch gekennzeichnet, daß sie parenteral verabreichbar ist.

5. Nahrungszusammensetzung nach Anspruch 3, dadurch gekennzeichnet, daß sie enteral verabreichbar ist.

6. Nahrungszusammensetzung nach Anspruch 5, dadurch gekennzeichnet, daß sie als Tierfutter gedacht ist.

7. Nahrungszusammensetzung nach Anspruch 5, dadurch gekennzeichnet, daß sie in flüssiger Form vorliegt.

8. Nahrungszusammensetzung nach Anspruch 5, dadurch gekennzeichnet, daß sie in Pulverform vorliegt, wobei die Triglyceride gekapselt oder mikrogekapselt sind.

**Revendications**

1. Les triglycérides 2-arachidoyl-1,3-di(octanoyl/décanoyl) glycérol.

2. Triglycérides selon la revendication 1 qui ont une pureté d'au moins 10 %, de préférence d'au moins 30 %, mieux d'au moins 50 %, encore mieux d'au moins 75 %, et tout préférablement d'au moins 90 %.

3. Composition nutritionnelle qui comprend les triglycérides selon la revendication 1 ou 2.

4. Composition nutritionnelle selon la revendication 3 qui peut être administrée par voie parentérale.

5. Composition nutritionnelle selon la revendication 3 qui peut être administrée par voie entérale.

6. Composition nutritionnelle selon la revendication 5 destinée à la nourriture des animaux.

7. Composition nutritionnelle selon la revendication 5 qui est sous une forme fluide.

8. Composition nutritionnelle selon la revendication 5 qui est sous forme d'une poudre, les triglycérides étant encapsulés ou microencapsulés.